# EUROPEAN PATENT SPECIFICATION

(11) **EP 3 120 907 B1**
(45) Date of publication and mention of the grant of the patent: **26.09.2018**
(21) Application number: 16180554.4
(22) Date of filing: 21.07.2016
(51) Int. Cl.: B01D 5/00, A61L 9/22, C02F 1/18, C02F 1/32, C02F 1/44, E03B 3/28, F24F 3/14

(54) **ATMOSPHERE DEW TRANSFORMATION SYSTEM**
ATMOSPHÄRENTAUTRANSFORMATIONSSYSTEM
SYSTÈME DE TRANSFORMATION DE ROSÉE DANS L'ATMOSPHÈRE

(30) Priority: 21.07.2015 CN 201510431527
(43) Date of publication of application: 25.01.2017
(73) Proprietor: NG, Tat Yung, North Point, Hong Kong (CN); NG, Tsz Yan Irys, North Point, Hong Kong (CN)
(72) Inventor: NG, Tat Yung, North Point, Hong Kong (CN); NG, Tsz Yan Irys, North Point, Hong Kong (CN)
(74) Representative: Michalski Hüttermann & Partner Patentanwälte mbB

(56) References cited:
- WO-A1-2012/009024
- CN-A- 103 256 675
- CN-A- 104 645 737
- US-A- 5 669 221
- US-A1- 2005 139 552

## Description

### TECHNICAL FIELD

The present invention relates to an atmosphere dew transformation system device, and particularly, to an air purification device and a water preparation device, and more particularly, to an air purification device part capable of releasing negative oxygen ions and a water preparation device part extracting high-quality, clean, sterile, nonhazardous acid-alkaline-neutral three-in-one drinkable water from air moisture.

### BACKGROUND

At present, the humankind is easier to utilize fresh water resources which are mainly from rivers, lakes and shallow groundwater. The fresh water resources in case of being used heavily are short; moreover, due to severe pollution and extremely unbalanced regional distribution, clean water resources are still lacked possibly even in a site having sufficient power and energy sources; therefore, the water resources are becoming a valuable resource, and the water resource issue even becomes a major strategy concerning national economy, social sustainable development and lasting political stability.

In some remote sites which are difficult to obtain clean water sources or lack of clean water sources, it is very difficult to pay a long-distance conveying pipeline for long-distance water supply to provide drinkable water, or convey the drinkable water back and forth through transportation vehicles regardless of transportation cost. These methods will make the water using cost become very high, so that common people cannot accept and enjoy the drinkable water necessary for life. Besides the high cost, water quality change or pollution may be possibly caused due to long-distance conveying or carrying, so that people cannot directly drink the water.

Moreover, people may not have a very high quantity demanded on the drinkable water (the quantity demanded is different according to different places, different climates and different environments), but have higher healthy, clean, sterile and nonhazardous requirements, wherein the requirements are substantially consistent. It becomes an urgent issue for national development to how to extract and prepare purified drinkable water (people consider this as dew) using the atmosphere (air) under reasonable costs in places having energy sources and power.

The atmosphere dew transformation system device may be applied to a water source and an air transformation system with small dosage for household purpose and big dosage for industrial purpose, which removes excessive water and atmospheric pollution components in the air, purifies the air and controls the indoor air humidity on one hand, and transforms excessive water in the air into drinkable sanitarian water, and transforms tap water or other undrinkable water into drinkable sanitarian water on the other hand, so that the indoor air and the drinkable water are improved at the same time. The atmosphere dew transformation system device is suitable for some families (children and old people, etc) having higher requirements on the water quality and air quality.
Document CN 103256675 A shows a air purification and drying apparatus comprising an air filter module with an air filter sleeve, an ultraviolet sterilization module with an ultraviolet lamp, a freeze-drying dehumidification module with an exhaust fan, a freeze dryer heat exchanger, a chilled water tank and a chilled water circulating water pump and a negative oxygen ion generation module with a negative oxygen ion generator.
Document WO 2012/009024 A1 discloses a system to extract water moisture from the atmosphere, highly purify and condition the extracted water, and provide beverages for human consumption. The system collects water-vapor condensation from filtered intake air, and then subjects the condensate to a series of purification filters and to a sterilization process in order to produce drinking water. The system may then use the water produced to provide drinks or ice.

### SUMMARY

The object of the present invention is to provide an atmosphere dew transformation system device, wherein the system includes an air purification device part capable of releasing negative oxygen ions, and a water preparation device part extracting high-quality, clean, sterile, nonhazardous acid-alkaline-neutral three-in-one drinkable water from air moisture. The air purification device regulates whether to enable a dehumidification function to prepare fresh air rich in negative oxygen ions according to the air humidity; the water preparation device regulates the water quantity according to the quantity of the air moisture, and has a very high water preparation amount in some ocean and inland climate regions with high humidity. In some regions which are polluted or have undrinkable water quality, the water may be purified into direct drinkable water through primary filtering and secondary filtering, sterilization and disinfection systems, wherein the purified water quantity may satisfy the demands of drinkable water for household purpose and industrial and commercial purposes.

To achieve the above object, the present invention employs a following technical solution.

The atmosphere dew transformation system device includes an air purification device part 1 and a water preparation device part 2.

The air purification device part 1 comprises a housing 11 and a filtering unit disposed in the housing, the filtering unit comprises a filter screen 12, a fiber cloth layer 13, an activated carbon net layer 14, a drive motor 15, an orifice plate 16, an impeller 17, an air outlet 18, an ultraviolet germicidal lamp 19 and a control panel 110; the left and right sides at the middle lower portion of the housing 11 are provided with housing openings, the filter screen 12 is disposed in the housing 11 and located at the front of the housing opening, the top end of the filter screen 12 is provided with the fiber cloth layer 13, the activated carbon net layer 14 is disposed above the fiber cloth layer 13 in a spacing manner, the filter screen 12, the fiber cloth layer 13, and the activated carbon net layer 14 are disposed parallel to each other and are fixedly connected to the inner wall of the housing 1, the orifice plate 16 is disposed above the activated carbon net layer 14, the orifice plate 16 is fixedly connected to the inner wall of the housing 1, the drive motor 15 for driving the impeller 17 is fixedly mounted on the orifice plate 16, an output shaft of the drive motor 15 is connected to the impeller 17, the top end of the housing 11 is provided with an exhaust plate 18, the ultraviolet germicidal lamp 19 is disposed on the inner wall of the housing 1 located between the exhaust plate 18 and the orifice plate 16, and the control panel 110 is disposed on the outer wall of a step of the housing 11.

The water preparation device part 2 includes a housing 21, a control panel 225 disposed on the housing, a humidity regulator 22, an air filter 23, a condensing evaporator 24, a water catching tray, a water catching tank 26, a water catching tray water level controller 27, a booster pump 28, a filtering system 29, a reverse osmosis purification system, a nanometer ultraviolet sterilization disinfector 211, a constant temperature water purification tank, an electrolytic bath 213, a drinkable acid water valve, a drinkable alkaline water valve 215, a drinkable neutral water valve 216, an exhauster 217, a compressor 220, an exhaust plate 223, a negative oxygen ion generator 226, and a tap water or undrinkable water source inlet 227.

The air purification device part 1 and the water preparation device part 2 are connected through a pipeline 00, and the pipeline 00 is connected between the exhaust plate 18 and the air filter 23

The working principle of the atmosphere dew transformation system device is as follows: the impeller 17 is controlled to rotate through the drive motor 15, air enters inside the housing 11 from the housing opening, then the filter screen 12, the fiber cloth layer 13 and the activated carbon net layer 14 process the air in sequence, the air after being processed, penetrates through the orifice plate 16 and then is disinfected by the ultraviolet germicidal lamp 19, and the purified air is exhausted to the air filter 23 by the exhaust plate 18 via the pipeline 00, an air moisture extraction system composed of the humidity regulator 22, the exhauster 217, the condensing evaporator 24 and the compressor reduces the temperature of the air to a dew point temperature through the condensing evaporator 24; under a dew point temperature, the air moisture is extracted and condensed into water globules, then the water globules automatically drop on the water catching tray 25 from the condensing evaporator; the water catching tray 25 collects the water into the water catching tank 26, the water catching tank 26 controls the booster pump 28 connected to the water catching tank to boost the water according to the water level controller 27, and the water flows to the filtering system 29 to remove generally mini impurities; then most inorganic salt (including heavy metal), organic matter, bacteria and virus dissolved in the water are separated by the reverse osmosis purification system 210 through adsorption hyperfiltration and ultrapurification, so that the water quality is directly transformed to that of ultrapure water; the ultrapure water is then connected to the nanometer ultraviolet sterilization disinfector 211, re-purified and then stored in a water purification tank, the inside of the purified water tank is provided with a water level display and the bottom of the purified water tank is respectively connected to the electrolyzed water supply valve 216 and the electrolytic bath 213 for a user to select to discharge neutral water or make the neutral water pass through the electrolytic bath 213, and acid water or alkaline water is discharged by the drinkable acid water valve 214 and the drinkable alkaline water valve 215; and purified and dehumidified air after passing through the air moisture extraction system composed of the humidity regulator 22, the exhauster 217, the condensing evaporator 24 and the compressor 220 of the water preparation device part 2, is discharged by the condensing evaporator 24 via the exhaust plate 223 after passing through the negative oxygen ion generator 226.

The humidity controller 22 controls whether the humidity of the primarily processed air discharged from the exhaust plate 18 of the air purification device part 1 is sufficient to extract the moisture in the air; if the air humidity is excessively low which is lower than 38%, then the moisture extraction function of the air moisture extraction system composed of the exhauster 217, the condensing evaporator 24 and the compressor 220 stops operation, and only the air temperature regulation function of the air moisture extraction system is used only. While the tap water or source for water not satisfying a drinkable standard 227 is connected to the filtering system 29, the reverse osmosis purification system 210 and the nanometer ultraviolet sterilization disinfector 211 through the booster pump 28 in the atmosphere dew transformation system device. The air purification device part 1 of the atmosphere dew transformation system device is connected to the outdoors, processing the air pumped from the outdoors, and discharges good-quality air which is purified and added with negative oxygen ions to the indoors through the water preparation device part 2.

The tap water or source for water not satisfying a drinkable standard 227 may be processed through a built-out primary filter, for example, a carbon particle candle filter and an activated carbon filter need to be added in case of a poorer water source, to filter the impurities in the water, and then the water is connected to the booster pump 28 of the water preparation device of the atmosphere dew transformation system.

The atmosphere dew transformation system device may filter the dust, soot, stive, cotton linters, animal dander, acarid, pollen, mold spore and germ-carrying particulates and the like; and control the air humidity and increase the negative oxygen ions through the water preparation device part 2, discharge clean air to the outdoors, and can also implement the function of regulating the air temperature at the same time; while the water preparation device part 2 may prepare high-quality, clean, sterile, nonhazardous acid-alkaline-neutral three-in-one drinkable water. The atmosphere dew transformation system device integrates the functions of an air conditioner, an air purifier, a purified water machine and an acid-alkaline water machine, and is an more advantageous device.

### BRIEF DESCRIPTION OF THE DRAWINGS

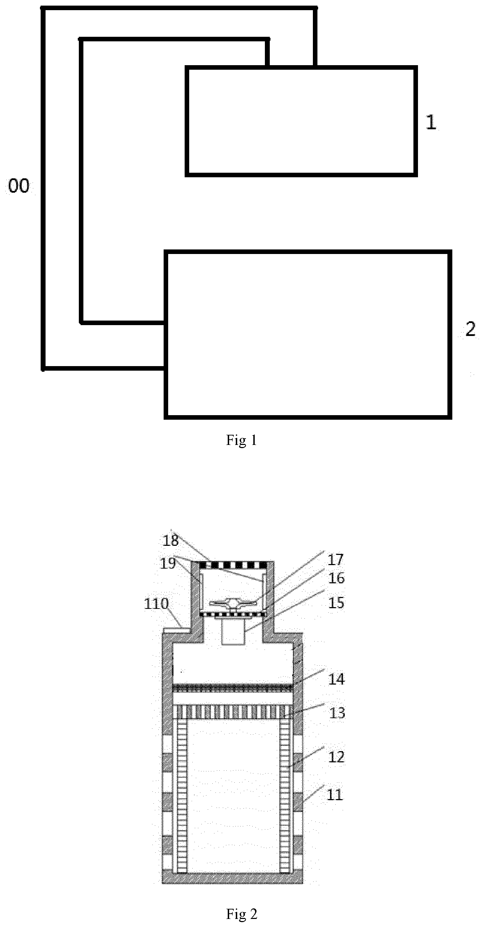

Fig. 1 is a block diagram of an atmosphere dew transformation system device.
   1 is an air purification device part, 2 is a water preparation device part, and the air purification device part 1 is connected to the water preparation device part 2 through a pipeline 00.
Fig. 2 is a block diagram of the air purification device part.

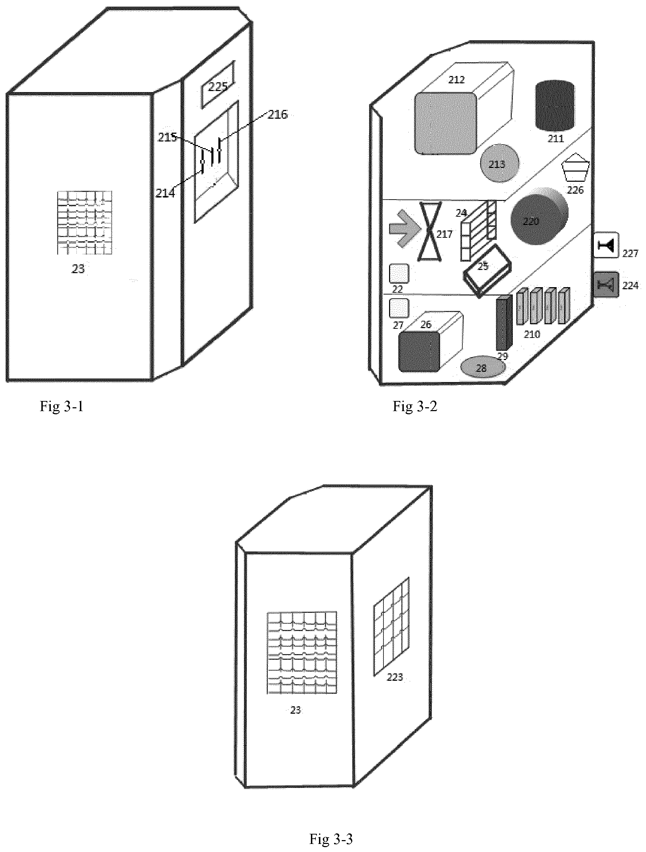

Fig. 3-1 is a front side block diagram of the water preparation device part.
Fig. 3-2 is an interior block diagram of the water preparation device part.
Fig. 3-3 is a back block diagram of the water preparation device part.

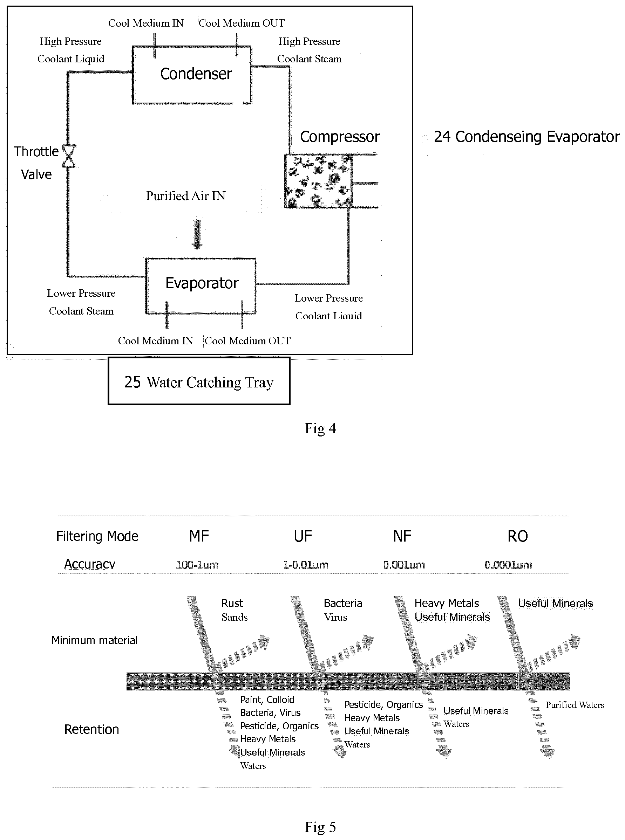

Fig. 4 is a working schematic diagram of a condensing evaporator 24.
Fig. 5 is a working flow and schematic diagram of a reverse osmosis purification system 210.

The main working principle of a reverse osmosis purification system is that a reverse osmosis membrane technology is employed. The working principle of the reverse osmosis purification system is that: a certain pressure is applied to water, so that water molecules and mineral substance elements in an ionic state pass through the reverse osmosis membrane, while most inorganic salt (including heavy metal), organic matter and bacteria, virus and the like cannot penetrate through the reverse osmosis membrane, so that osmosed purified water and concentrated water which cannot be osmosed are strictly separated.

An aperture on the reverse osmosis membrane is 0.0001 micron only, while the diameter of the virus is generally 0.02-0.4 micron, and the diameter of common bacteria is only 0.4-1; therefore, the pure water flowing out from a purified water machine may be drunk securely.

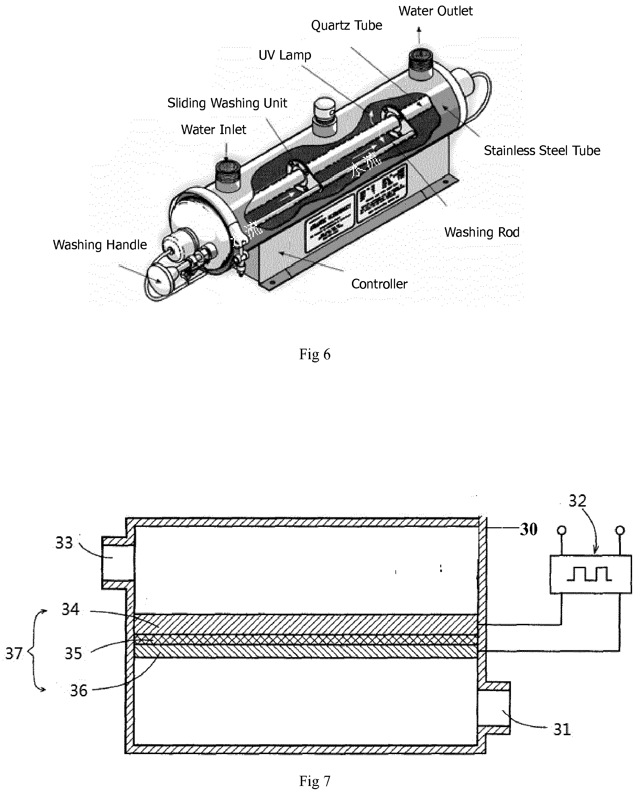

Fig. 6 is a working schematic diagram of a nanometer ultraviolet sterilization disinfector 211.

A barrel of a nanometer ultraviolet ray disinfector is manufactured using stainless steel, most of the inner wall thereof is polished so as to improve the ultraviolet reflectivity and enhance the radiation intensity; moreover, the number of ultraviolet lamps may be regulated according to the amount of water to be processed. The inner wall of the barrel of some disinfector is additionally provided with a spiral blade so as to change the movement state of a water flow, thus avoiding dead water and pipeline plugging; a turbulent flow produced and the sharp edge of the blade will shatter suspended solids, so that adherent microorganisms are completely exposed in ultraviolet irradiation, thus improving the disinfection efficiency.

The disinfection principle of the nanometer ultraviolet sterilization disinfector is to mainly utilize ultraviolet rays having a wavelength of 254 nanometers. The ultraviolet rays with this wavelength may destroy the life core of one cell-DNA even in case of a trace ultraviolet ray projection dosage; therefore, the cell is kept from regeneration, and loses regenerative power, so that the bacteria become innocuous, thus achieving the effect of sterilization. Like all other ultraviolet ray application technologies, the scale of this system is depended on the ultraviolet intensity (the intensity and power of an irradiator) and contact time (time duration under water, liquid or air exposed under ultraviolet rays).

Fig. 7 is a working schematic diagram of a negative oxygen ion generator 226.

The negative ion generator 226 is composed of a housing 30 as well as a foamed nickel net first electrode 34 fixedly mounted on the inner surface of the housing covering nanometer titanium oxide, a gauze insulating layer 35, a stainless steel net second electrode 36, and a voltage converting circuit 32. The first electrode 34, the gauze insulating layer 35 and the second electrode 36 are installed cling to each other to form a component 37 having capacitance characteristics. The component 37 and the housing 30 are tightly contacted at a contact surface for installation and fixation to prevent air leakage. An air inlet 31 and an air outlet 33 are disposed on the housing 30. The air flows in from the air inlet 31, flows through the first electrode 34, the gauze insulating layer 35 and the second electrode 36 in sequence, and flows out from the air outlet 33. The negative oxygen ion generator is charged by a 12V storage battery (not shown in the figure). The working principle of the negative oxygen ion generator is that: the air enters the inside of the negative oxygen ion generator from the air inlet 31, wherein redox reaction occurs to the oxygen therein while being contacted with the first electrode 34 to generate negative oxygen ions, and the negative oxygen ions flow out from the air outlet 33 together with an air flow.

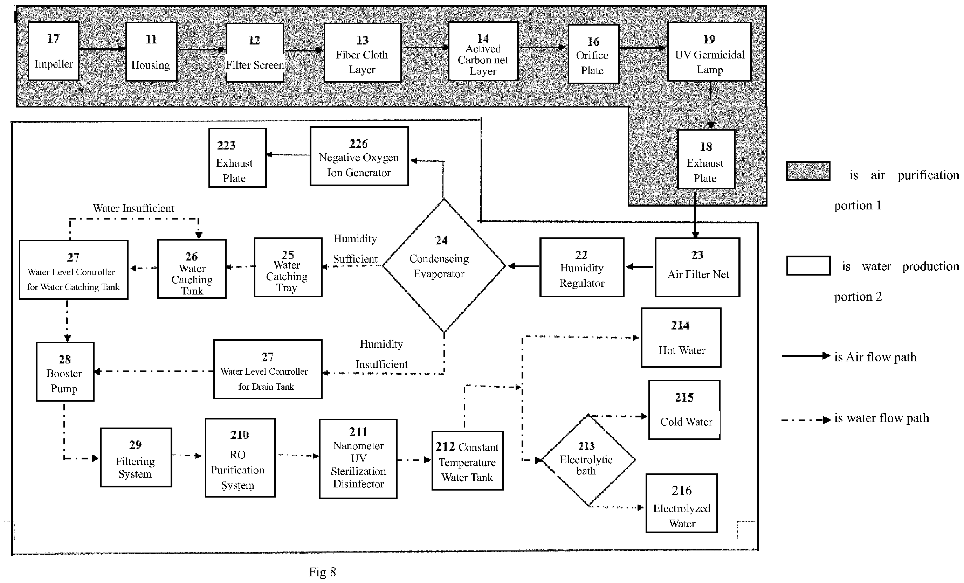

Fig. 8 is a working flow chart of an atmosphere dew transformation system device.

### DETAILED DESCRIPTION

### Embodiment 1

The working principle of an atmosphere dew transformation system device is, there is a certain humidity in the air..

An impeller 17 is controlled to rotate through a drive motor 15, air enters inside a housing 11 from a housing opening, then a filter screen 12, a fiber cloth layer 13 and an activated carbon net layer 14 process the air in sequence, the air after being processed, penetrates through an orifice plate 16 and then is disinfected by an ultraviolet germicidal lamp 19, and the purified air is exhausted to an air filter 23 by an exhaust plate 18 via a pipeline 00.

A humidity regulator 22 detects that the humidity of the air entered from an air filter 23 is higher than 55% which is 65%, then an air moisture extraction system composed of the humidity regulator 22, an exhauster 217, a condensing evaporator 24, and a compressor starts working, and the temperature of the air is reduced to a dew point temperature by a condensing evaporator 24. Under a dew point temperature, the air moisture is extracted and condensed into water globules, then the water globules automatically drop on a water catching tray 25 from the condensing evaporator; the water catching tray 25 collects the water into a water catching tank 26, the water catching tank 26 controls a booster pump 28 connected to the water catching tank to boost the water according to a water level controller 27, and the water flows to a filtering system 29 to remove generally mini impurities; then most inorganic salt (including heavy metal), organic matter, bacteria and virus dissolved in the water are separated by a reverse osmosis purification system 210 through adsorption hyperfiltration and ultrapurification, so that the water quality is directly transformed to that of ultrapure water; the ultrapure water is then connected to a nanometer ultraviolet sterilization disinfector 211, re-purified and then stored in a water purification tank, the inside of the purified water tank is provided with a water level display and the bottom of the purified water tank is respectively connected to a drinkable neutral water valve 216 and an electrolytic bath 213 for a user to select to discharge neutral water or make the neutral water pass through the electrolytic bath 213, and acid water or alkaline water is discharged by a drinkable acid water valve 214 and a drinkable alkaline water valve 215; and purified and dehumidified air after passing through the air moisture extraction system composed of the humidity regulator 22, the exhauster 217, the condensing evaporator 24 and the compressor 220 of a water preparation device part 2, is discharged via an exhaust plate 223 after passing through the negative oxygen ion generator 226.

### Embodiment 2

Working of an atmosphere dew transformation system device in case of normal or excessively low air humidity

An impeller 17 is controlled to rotate through a drive motor 15, air enters inside a housing 11 from a housing opening, then a filter screen 12, a fiber cloth layer 13 and an activated carbon net layer 14 process the air in sequence, the air after being processed, penetrates through an orifice plate 16 and then is disinfected by an ultraviolet germicidal lamp 19, and the purified air is exhausted to an air filter 23 by an exhaust plate 18 via a pipeline 00.

A humidity regulator 22 detects that the humidity of the air entered from an air filter 23 is less than 38% which is 35%, then an air moisture extraction system composed of the humidity regulator 22, an exhauster 217, a condensing evaporator 24, and a compressor stops extracting moisture, but regulates the temperature of the air, and the temperature of the air is reduced to a proper temperature by a condensing evaporator 24.
a tap water or source for water not satisfying a drinkable standard 227 after being re-purified by a filtering system 29, a reverse osmosis purification system 210 and a nanometer ultraviolet sterilization disinfector 211 through a booster pump 28 in the atmosphere dew transformation system device, is stored in a water purification tank 212; and the inside of a purified water tank is provided with a water level display and the bottom of the purified water tank is respectively connected to the electrolyzed water supply valve 216 and the electrolytic bath 213 for a user to select to discharge neutral water or make the neutral water pass through the electrolytic bath 213, and acid water or alkaline water is discharged by a drinkable acid water valve 214 and a drinkable alkaline water valve 215. and purified and dehumidified air after passing through the air moisture extraction system composed of the humidity regulator 22, the exhauster 217, the condensing evaporator 24 and the compressor 220 of a water preparation device part 2, is discharged via an exhaust plate 223 after passing through the negative oxygen ion generator 226.

## Claims

1. An atmosphere dew transformation system device, wherein the atmosphere dew transformation system device comprises an air purification device part (1) and a water preparation device part (2);
the air purification device part (1) comprises a housing (11) and a filtering unit disposed in the housing,
the filtering unit comprises a filter screen (12), a fiber cloth layer (13), an activated carbon net layer (14), a drive motor (15), an orifice plate (16), an impeller (17), an air outlet (18), an ultraviolet germicidal lamp (19) and a control panel (110);
the water preparation device part (2) comprises a housing (21), a condensing evaporator (24), a water catching tank (26), a booster pump (28), an exhauster (217), an exhaust plate (223), and a negative oxygen ion generator (226),
**characterized in that**
the left and right sides at the middle lower portion of the housing (11) are provided with housing openings, the filter screen (12) is disposed in the housing (11) and located at the front of the housing opening, the top end of the filter screen (12) is provided with the fiber cloth layer (13), the activated carbon net layer (14) is disposed above the fiber cloth layer (13) in a spacing manner, the filter screen (12), the fiber cloth layer (13), and the activated carbon net layer (14) are disposed parallel to each other and are fixedly connected to the inner wall of the housing (1), the orifice plate (16) is disposed above the activated carbon net layer (14), the orifice plate (16) is fixedly connected to the inner wall of the housing (1), the drive motor (15) for driving the impeller (17) is fixedly mounted on the orifice plate (16), an output shaft of the drive motor (15) is connected to the impeller (17), the top end of the housing (11) is provided with an exhaust plate (18), the ultraviolet germicidal lamp (19) is disposed on the inner wall of the housing (1) located between the exhaust plate (18) and the orifice plate (16), and the control panel (110) is disposed on the outer wall of a step of the housing (11);
the water preparation device part (2) further comprises a control panel (225) disposed on the housing, a humidity regulator (22), an air filter (23), a water catching tray, a water catching tray water level controller (27), a filtering system (29), a reverse osmosis purification system (210), a nanometer ultraviolet sterilization disinfector (211), a constant temperature water purification tank (212), a valve (216) for drinkable neutral water, an electrolytic bath (213), a valve (214) for drinkable acid water, a valve (215) for drinkable alkaline water, , a compressor (220), and a tap water or undrinkable water source inlet;
the air purification device part (1) and the water preparation device part (2) are connected through a pipeline (00), and the pipeline (00) is connected between the exhaust plate (18) and the air filter (23); the atmosphere dew transformation system device is configured in such way that the working principle of the atmosphere dew transformation system device is that: the impeller (17) is controlled to rotate through the drive motor (15), air enters inside the housing (11) from the housing opening, then the filter screen (12), the fiber cloth layer (13) and the activated carbon net layer (14) process the air in sequence, the air after being processed, penetrates through the orifice plate (16) and then is disinfected by the ultraviolet germicidal lamp (19), and the purified air is exhausted to the air filter (23) by the exhaust plate (18) via the pipeline (00);
an air moisture extraction system composed of the humidity regulator (22), the exhauster (217), the condensing evaporator (24) and the compressor reduces the temperature of the air to a dew point temperature through the condensing evaporator (24); under the dew point temperature, the air moisture is extracted and condensed into water globules, then the water globules automatically drop on the water catching tray (25) from the condensing evaporator (24);
the water catching tray (25) collects the water into the water catching tank (26), the water catching tank (26) controls the booster pump (28) connected to the water catching tank (26) to boost the water according to the water level controller (27), and the water after being re-purified by the filtering system (29), the reverse osmosis purification system (210) and the nanometer ultraviolet sterilization disinfector (211) is stored in the constant temperature water purification tank (212); and
the inside of a purified water tank (212) is provided with a water level display and the bottom of the purified water tank (212) is respectively connected to the electrolyzed water supply valve (216) and the electrolytic bath (213) for a user to select to discharge neutral water or make the neutral water pass through the electrolytic bath (213), and acid water or alkaline water is discharged by the hot water supply valve (214) and the cold water supply valve (215); and purified and dehumidified air after passing through the air moisture extraction system composed of the humidity regulator (22), the exhauster (217), the condensing evaporator (24) and the compressor (220) of the water preparation device part (2), is discharged by the condensing evaporator (24) via the exhaust plate (223) after passing through the negative oxygen ion generator (226).

2. The atmosphere dew transformation system device according to claim 1, wherein the device further comprises a tap water source or a source for water not satisfying a drinkable standard (227); when the humidity regulator (22) detects that the humidity is lower than 38%, the tap water or source for water not satisfying a drinkable standard (227) is connected to the filtering system (29), the reverse osmosis purification system (210) and the nanometer ultraviolet sterilization disinfector (211) through the booster pump (28) in the atmosphere dew transformation system device

3. The atmosphere dew transformation system device according to claim 1, wherein the negative oxygen ion generator (226) is composed of a housing (30) as well as a foamed nickel net first electrode (34) fixedly mounted on the inner surface of the housing covering nanometer titanium oxide, a gauze insulating layer (35), a stainless steel net second electrode (36), and a voltage converting circuit (32), the first electrode (34), the gauze insulating layer (35) and the second electrode (36) are installed cling to each other to form a component (37) having capacitance characteristics, the component (37) and the housing (30) are tightly contacted at a contact surface for installation and fixation to prevent air leakage, an air inlet (31) and an air outlet (33) are disposed on the housing (30), the air flows in from the air inlet (31), flows through the first electrode (34), the gauze insulating layer (35) and the second electrode (36) in sequence, and flows out from the air outlet (33), the negative oxygen ion generator is charged by a 12V storage battery, and the working principle of the negative oxygen ion generator is that: the air enters the inside of the negative oxygen ion generator from the air inlet (31), wherein redox reaction occurs to the oxygen therein while being contacted with the first electrode (34) to generate negative oxygen ions, and the negative oxygen ions flow out from the air outlet (33) together with an air flow.

## Patentansprüche

1. Systemeinheit zum Umwandeln von Tau aus der Atmosphäre, wobei
die Systemeinheit zum Umwandeln von Tau aus der Atmosphäre einen Luftreinigungseinrichtungsteil (1) und einen Wasseraufbereitungseinrichtungsteil (2) aufweist,
der Luftreinigungseinrichtungsteil (1) ein Gehäuse (11) und eine im Gehäuse angeordnete Filtereinheit aufweist,
die Filtereinheit ein Filtersieb (12), eine Fasergewebeschicht (13), eine Aktivkohlenetzschicht (14), einen Antriebsmotor (15), eine Lochplatte (16), ein Flügelrad (17), einen Luftauslass (18), eine UV-Entkeimungslampe (19) und ein Bedienfeld (110) aufweist,
der Wasseraufbereitungseinrichtungsteil (2) ein Gehäuse (21), einen Kondensationsverdampfer (24), einen Wasserauffangbehälter (26), eine Druckerhöhungspumpe (28), einen Luftabzug (217), eine Auslassplatte (223) und einen Generator (226) für negative Sauerstoffionen aufweist;
**dadurch gekennzeichnet, dass**
die linke und die rechte Seite am mittleren unteren Bereich des Gehäuses (11) mit Gehäuseöffnungen versehen sind, das Filtersieb (12) im Gehäuse (11) an der Vorderseite der Gehäuseöffnung angeordnet ist, die Fasergewebeschicht (13) am oberen Ende des Filtersiebs (12) angeordnet ist, die Aktivkohlenetzschicht (14) oberhalb der Fasergewebeschicht (13) und beabstandet davon angeordnet ist, das Filtersieb (12), die Fasergewebeschicht (13) und die Aktivkohlenetzschicht (14) parallel zueinander angeordnet und fest mit der Innenwand des Gehäuses (1) verbunden sind, die Lochplatte (16) oberhalb der Aktivkohlenetzschicht (14) angeordnet ist, die Lochplatte (16) fest mit der Innenwand des Gehäuses (1) verbunden ist, der Antriebsmotor (15) zum Antreiben des Flügelrades (17) fest an der Lochplatte (16) befestigt ist, eine Abtriebswelle des Antriebsmotors (15) mit dem Flügelrad (17) verbunden ist, die Auslassplatte (18) am obere Ende des Gehäuses (11) angeordnet ist, die UV-Entkeimungslampe (19) an der Innenwand des Gehäuses (1) zwischen der Auslassplatte (18) und der Lochplatte (16) angeordnet ist, und das Bedienfeld (110) an der Außenwand einer Stufe des Gehäuses (11) angeordnet ist;
der Wasseraufbereitungseinrichtungsteil (2) ferner aufweist:
ein am Gehäuse angeordnetes Bedienfeld (225), einen Feuchtigkeitsregler (22), einen Luftfilter (23), eine Wasserauffangschale, eine Wasserauffangschalenwasserstandssteuereinheit (27), ein Filtersystem (29), ein Umkehrosmose-Reinigungssystem (210), einen Nanometer-UV-SterilisationsDesinfektor (211), einen Konstanttemperatur-Wasserreinigungstank (212), ein Ventil (216) für trinkbares neutrales Wasser, ein Elektrolytbad (213), ein Ventil (214) für trinkbares saures Wasser, ein Ventil (215) für trinkbares alkalisches Wasser, einen Kompressor (220) und einen Einlass für eine Leitungswasserquelle oder für eine Quelle für nicht trinkbares Wasser;
der Luftreinigungseinrichtungsteil (1) und der Wasseraufbereitungseinrichtungsteil (2) durch eine Rohrleitung (00) verbunden sind, und die Rohrleitung (00) zwischen der Auslassplatte (18) und dem Luftfilter (23) verbunden ist;
die Systemeinheit zum Umwandeln von Tau aus der Atmosphäre derart konfiguriert ist, dass das Arbeitsprinzip der Systemeinheit zum Umwandeln von Tau aus der Atmosphäre darin besteht, dass: das Flügelrad (17) derart gesteuert wird, dass es sich durch den Antriebsmotor (15) dreht, Luft über die Gehäuseöffnung in das Gehäuse (11) eintritt, dann das Filtersieb (12), die Fasergewebeschicht (13) und die Aktivkohlenetzschicht (14) die Luft nacheinander verarbeiten, die Luft, nachdem sie verarbeitet worden ist, die Lochplatte (16) durchströmt und dann durch die UV-Entkeimungslampe (19) desinfiziert wird, und die gereinigte Luft durch die Auslassplatte (18) und über die Rohrleitung (00) zum Luftfilter (23) ausgegeben wird;
ein Luftfeuchtigkeitsextraktionssystem, das aus dem Feuchtigkeitsregler (22), dem Luftabzug (217), dem Kondensationsverdampfer (24) und dem Kompressor besteht, die Temperatur der Luft durch den Kondensationsverdampfer (24) auf eine Taupunkttemperatur reduziert, die Luftfeuchtigkeit unter der Taupunkttemperatur extrahiert und in Wasserkügelchen kondensiert wird und die Wasserkügelchen dann automatisch vom Kondensationsverdampfer (24) auf die Wasserauffangschale (25) fallen;
die Wasserauffangschale (25) das Wasser in den Wasserauffangbehälter (26) sammelt, der Wasserauffangbehälter (26) eine mit dem Wasserauffangbehälter (26) verbundene Druckerhöhungspumpe (28) steuert, um den Wasserdruck gemäß der Wasserstandssteuereinheit (27) zu erhöhen, und das Wasser, nachdem es durch das Filtersystem (29), das Umkehrosmose-Reinigungssystem (210) und den Nanometer-UV-SterilisationsDesinfektor (211) erneut gereinigt wurde, im Konstanttemperatur-Wasserreinigungstank (212) gespeichert wird; und
im Inneren eines Reinwasserbehälters (212) eine Wasserstandsanzeige vorgesehen ist und der Boden des Reinwasserbehälters (212) jeweils mit dem Elektrolysewasserzufuhrventil (216) und dem Elektrolysebad (213) verbunden ist, so dass ein Benutzer die Wahl hat, neutrales Wasser abzulassen oder das neutrale Wasser durch das Elektrolysebad (213) laufen zu lassen, um saures Wasser oder alkalisches Wasser durch das Heißwasserzufuhrventil (214) und das Kaltwasserzufuhrventil (215) auszugegeben, und gereinigte und entfeuchtete Luft, nachdem sie das Luftfeuchtigkeitsextraktionssystems, das aus dem Feuchtigkeitsregler (22), dem Luftabzug (217), dem Kondensationsverdampfer (24) und dem Kompressor (220) des Wasseraufbereitungseinrichtungsteils (2) besteht, durch den Kondensationsverdampfer (24) über die Auslassplatte (223) ausgegeben wird, nachdem sie den Generator (226) für negative Sauerstoffionen (226) durchlaufen hat.

2. Systemeinheit nach Anspruch 1, wobei die Einheit ferner eine Leitungswasserquelle oder eine Quelle für Wasser aufweist, das keinen trinkbaren Standard (227) erfüllt, wobei, wenn der Feuchtigkeitsregler (22) erfasst, dass die Feuchtigkeit niedriger ist als 38%, das Leitungswasser oder eine Quelle für Wasser, das keinen trinkbaren Standard (227) erfüllt, durch die Druckerhöhungspumpe (28) der Systemeinheit zum Umwandeln von Tau aus der Atmosphäre mit dem Filtersystem (29), dem Umkehrosmose-Reinigungssystem (210) und dem Nanometer-UV-Sterilisations-Desinfektor (211) verbunden wird.

3. Systemeinheit nach Anspruch 1, wobei der Generator (226) für negative Sauerstoffionen aus einem Gehäuse (30) sowie einer ersten Elektrode (34) aus einem geschäumten Nickelnetz, die fest an der mit Nanometer-Titanoxid bedeckten Gehäuseinnenfläche montiert ist, einer Gaze-Isolierschicht (35), einer zweiten Elektrode (36) aus einem rostfreien Stahlnetz und einer Spannungsumwandlungsschaltung (32) besteht, wobei die erste Elektrode (34), die Gaze-Isolierschicht (35) und die zweite Elektrode (36) aneinander befestigt sind, um eine Komponente (37) mit Kapazitätseigenschaften zu bilden, wobei die Komponente (37) und das Gehäuse (30) an einer Kontaktfläche zur Installation und Fixierung eng in Kontakt stehen, um ein Entweichen von Luft zu verhindern, ein Lufteinlass (31) und ein Luftauslass (33) auf dem Gehäuse (30) angeordnet sind, die Luft über den Lufteinlass (31) zuströmt, die erste Elektrode (34), die Gaze-Isolierschicht (35) und die zweite Elektrode (36) nacheinander durchströmt und aus dem Luftauslass (33) ausströmt, der Generator für negative Sauerstoffionen durch eine 12 V-Speicherbatterie geladen wird, und das Arbeitsprinzip des Generators für negative Sauerstoffionen darin besteht, dass die Luft über den Lufteinlass (31) in das Innere des Generators für negative Sauerstoffionen eintritt, in dem eine Redoxreaktion bezüglich des Sauerstoffs stattfindet, während sie mit der ersten Elektrode (34) in Kontakt kommt, um negative Sauerstoffionen zu erzeugen, und wobei die negativen Sauerstoffionen zusammen mit einer Luftströmung aus dem Luftauslass (33) ausströmen.

## Revendications

1. Unité de système de transformation de la rosée atmosphérique, dans lequel l'unité de système de transformation de rosée atmosphérique comprend une partie d'unité de système de purification d'air (1) et une partie d'unité de système de préparation d'eau (2) ;
la partie d'unité de système de purification d'air (1) comprend un boîtier (11) et une unité de filtration disposée dans le boîtier,
l'unité de filtration comprend un tamis (12), une couche de tissu de fibre (13), une couche de réseau de charbon actif (14), un moteur d'entraînement (15), une plaque à orifice (16), une turbine (17), une sortie d'air (18), une lampe germicide UV (19) et un panneau de commande (110) ;
la partie d'unité de système de préparation d'eau (2) comprend un boîtier (21), un évaporateur condensateur (24), un réservoir de récupération d'eau (26), une pompe de gavage (28), un échappement (217), une plaque d'échappement (223), et un générateur d'ion oxygène négatif (226),
**caractérisé en ce que**,
les côtés gauche et droit au niveau de la partie médiane inférieure du boîtier (11) sont fournis avec des ouvertures de boîtier, le tamis (12) est disposé dans le boîtier (11) et situé devant l'ouverture de boîtier, l'extrémité supérieure du tamis (12) est fournie avec la couche de tissu de fibre (13), la couche de réseau de charbon actif (14) est disposée au-dessus de la couche de tissu de fibre (13) de manière espacée, le tamis (12), la couche de tissu de fibre (13), et la couche de réseau de charbon actif (14) sont disposés de façon parallèle entre eux et sont reliés de manière fixe à la paroi interne du boîtier (1), la plaque à orifice (16) est disposée au-dessus de la couche de réseau de charbon actif (14), la plaque à orifice (16) est reliée de manière fixe à la paroi interne du boîtier, le moteur d'entraînement (15) pour entraîner la turbine (17) est relié de manière fixe à la plaque à orifice (16), un arbre de sortie du moteur d'entraînement (15) est relié à la turbine (17), l'extrémité supérieure du boîtier est fournie avec une plaque d'échappement (18), la lampe germicide UV (19) est disposée sur la paroi interne du boîtier (1) située entre la plaque d'échappement (18) et la plaque à orifice (16), et le panneau de commande (110) est disposé sur la paroi externe d'un créneau du boîtier (11) ;
la partie d'unité de système de préparation d'eau (2) comprend en outre un panneau de commande (225) disposé sur le boîtier, un régulateur d'humidité (22), un filtre à air (23), un plateau réceptionneur d'eau, un régulateur de niveau de plateau réceptionneur d'eau (27), un système de filtre (29), un système de purification à osmose inverse (210), un désinfectant par stérilisation UV nanométrique (211), un réservoir de purification d'eau à température constante (212), une valve (216) pour l'eau neutre potable, un bain électrolytique (213), une valve (214) pour l'eau acide potable, une valve (215) pour l'eau alcaline potable, un compresseur (220), et une entrée d'eau du robinet ou d'eau non-potable ;
la partie d'unité de système de purification d'air (1) et la partie d'unité de système de préparation d'eau (2) sont reliées par un pipeline (00) et le pipeline (00) est connecté entre la plaque d'échappement (18) et le filtre à air (23) ;
l'unité de système de transformation de la rosée atmosphérique est configurée de telle façon que
le principe de fonctionnement du système de transformation de la rosée atmosphérique est que : la turbine (17) est commandée de manière à tourner via le moteur d'entraînement (15), l'air entre à l'intérieur du boîtier (11) par l'ouverture du boîtier, ensuite le tamis (12), la couche de tissu de fibre (13) et la couche de réseau de charbon actif (14) traitent l'air de façon séquentielle, l'air une fois traité, pénètre à travers la plaque à orifice (16) et est ensuite désinfecté par la lampe germicide UV (19), et l'air purifié s'échappe vers le filtre à air (23) par la plaque d'échappement (18) via le pipeline (00) ;
un système d'extraction de l'humidité de l'air composé du régulateur d'humidité (22), de l'échappement (217), de l'évaporateur condensateur (24) et du compresseur, réduit la température de l'air à une température de point de rosée via l'évaporateur condensateur (24) ; au-dessous de la température de point de rosée, l'humidité de l'air est extraite et condensée en globules d'eau, ensuite les globules d'eau provenant de l'évaporateur condensateur (24) se déposent automatiquement sur le plateau réceptionneur d'eau (25) ;
le plateau réceptionneur d'eau (25) collecte l'eau dans le réservoir de récupération d'eau (26), le réservoir de récupération d'eau (26) contrôle la pompe de gavage (28) connectée au réservoir de récupération d'eau (26) pour appointer l'eau selon le régulateur de niveau d'eau (27), et l'eau une fois re-purifiée par le système de filtration (29), le système de purification à osmose inverse (210) et le désinfectant par stérilisation UV nanométrique (211) est stockée dans le réservoir de purification d'eau à température constante (212) ; et
l'intérieur d'un réservoir d'eau purifiée (212) est fourni avec un affichage du niveau d'eau et la partie inférieure du réservoir d'eau purifiée (212) est respectivement connectée à la valve d'alimentation d'eau électrolysée (216) et le bain électrolytique (213) est conçu de sorte que l'utilisateur puisse sélectionner le déversement d'eau neutre ou le passage de l'eau neutre par le bain électrolytique (213), et l'eau acide ou l'eau alcaline est déversée par la valve d'alimentation d'eau chaude (214) et la valve d'alimentation d'eau froide (215) ; et l'air déshumidifié et purifié après passage à travers le système d'extraction de l'humidité de l'air composé du régulateur d'humidité (22), de l'échappement (217), de l'évaporateur condensateur (24) et du compresseur (220) de la partie d'unité de système de préparation d'eau (2), est déversé par l'évaporateur condensateur (24) via la plaque d'échappement (223) après passage à travers le générateur d'ion oxygène négatif (226).

2. Unité de système de transformation de la rosée atmosphérique selon la revendication 1, dans lequel l'unité de système comprend une source d'eau du robinet ou une source d'eau ne satisfaisant pas aux critères d'eau potable (227) ; quand le régulateur d'humidité (22) détecte que l'humidité est plus basse que 38%, l'eau du robinet ou la source d'eau ne satisfaisant pas aux critères d'eau potable (227) est reliée au système de filtres (29), au système de purification à osmose inverse (210) et au désinfectant par stérilisation UV nanométrique (211) par la pompe de gavage (28) dans l'unité de système de transformation de la rosée atmosphérique.

3. Unité de système de transformation de la rosée atmosphérique selon la revendication 1, dans lequel le générateur d'ion oxygène négatif (226) est composé d'un boîtier (30) ainsi que d'une première électrode à réseau de nickel expansé montée de manière fixe à la surface interne du boîtier couvrant du dioxyde de titane nanométrique, une couche de gaze isolante (35), une seconde électrode à réseau d'acier inoxydable (36), et un circuit convertisseur de tension (32), la première électrode (34), la couche de gaze isolante (35) et la seconde électrode (36) sont installées de sorte à être attachées les unes aux autres pour former un composant (37) ayant des caractéristiques de capacitance, le composant (37) et le boîtier (30) sont en contact étroit à une surface de contact pour l'installation et la fixation en vue d'éviter les fuites d'air, une entrée d'air (31) et une sortie d'air (33) sont disposées sur le boîtier (30), l'air s'écoule vers l'intérieur par l'entrée d'air (31), s'écoule à travers la première électrode (34), la couche de gaze isolante (35) et la seconde électrode (36) de façon séquentielle, et s'écoule vers l'extérieur par la sortie d'air (33), le générateur d'ion oxygène négatif est chargé par un accumulateur 12V, et le principe de fonctionnement du générateur d'ion oxygène négatif est que : l'air entre à l'intérieur du générateur d'ion oxygène négatif par l'entrée d'air (31), dans lequel une réaction redox impliquant l'oxygène a lieu lorsqu'il est mis en contact avec la première électrode (34) pour générer des ions oxygènes négatifs, et les ions oxygènes négatifs s'écoulent vers l'extérieur par la sortie d'air (33) en même temps qu'un écoulement d'air.
